# EUROPEAN PATENT SPECIFICATION

(11) **EP 3 988 065 B1**
(45) Date of publication and mention of the grant of the patent: **28.08.2024**
(21) Application number: 21204288.1
(22) Date of filing: 22.10.2021
(51) Int. Cl.: A61F 13/15

(54) **COMPOSITE ABSORBENT STRUCTURE**
ABSORBIERENDE VERBUNDSTRUKTUR
STRUCTURE ABSORBANTE COMPOSITE

(30) Priority: 22.10.2020 IT 202000025078
(43) Date of publication of application: 27.04.2022
(73) Proprietor: FARE' S.p.A., 21054 Fagnano Olona (VA) (IT)
(72) Inventor: FARE', Rosaldo, 21054 Fagnano Olona (VA) (IT)
(74) Representative: Gislon, Gabriele

(56) References cited:
- EP-A1- 3 321 407
- US-A1- 2012 276 239
- US-A1- 2019 053 960
- US-B2- 6 703 846

## Description

### Field of the Invention

The present invention relates to the field of composite structures for applications such as industrial absorbents (cloths, rolls, pillows, mats, etc.), diapers and similar garments, for children and adults, having a plurality of layers, including a central absorbent layer.

### Prior art

Absorbent composite structures having a central layer typically comprising superabsorbent polymer (known in the art as SAP) with cellulose, typically a mixture of them in powder form, are known in the art.

However, since the cellulose has to be powdered during production, the production of the cellulose portion is complex. This process is particularly noisy and involves the production of powders dispersed in the environment with obvious damage to the health of the operators and, in general, of the people who breathe these powders. Additionally, the composite produced in this way is very thick due to the high amount of cellulose required to incorporate the SAP.

Methods of producing absorbent composite structures by incorporating cellulose and/or SAP with filaments/fibrils obtained by meltblown process are also known in the art. The composite structure so obtained, however, has low mechanical properties such as tensile strength and elongation and a low liquid-absorption rate due to the small diameter of the filaments/fibrils typically produced with the meltblown process that, acting somehow as a barrier, hamper the passage of the liquid. Furthermore, high production costs due to high power consumption, together with low productivity, are typical of meltblown technology.

US 2019/0053960 describes diaper implementations with spunbond nonwoven crimped fabric, i.e. curled, wherein the filaments are curled in the cooling step, before forming the nonwoven fabric. Napkins thus obtained may be free of cellulose powder, but in that case they often have gel-blocking problems, in particular if there are SAP particles with dimensions smaller than 100 µm (micron), see for example paragraph [0039] of US'960. In the case of gel-blocking, the liquid reaching the diaper cannot distribute through the non-woven fabric and Sap structure and there is a return to the outside of the liquid penetrated in the diaper.

Therefore, it is an object of the present invention to make an absorbent composite structure provided with good absorbent properties and good mechanical properties such as flexibility, tensile strength and elongation, in a more economic way than known and avoiding or limiting the use of cellulose. It is also an object of the present invention to make a composite absorbent structure having not only a good liquid absorption rate with maintenance of integrity when wet, but also allowing, at the same time, a thickness reduction for the same absorbent capacity. The structure of the present invention advantageously prevents or reduces gel blocking phenomena by the absorbent polymer.

These and other objects are solved by the present invention as hereinafter described.

### Summary of the invention

The present invention relates to a process, a composite structure and an apparatus according to the appended independent claims. Preferred aspects are set forth in the dependent claims.

According to an aspect of the present invention, a process for making a composite absorbent structure comprises the steps of: a. Feeding, from a first source onto a deposit surface, a first layer comprising a first nonwoven fabric b. Feeding superabsorbent polymer onto the first layer; c. Feeding, from a second source onto the assembly formed by the first layer and the superabsorbent polymer, a second layer comprising a second nonwoven fabric; d. Bonding the first nonwoven fabric and the second nonwoven fabric to each other by at least one calender. The first nonwoven fabric and/or the second nonwoven fabric comprises, preferably is basically composed of, multicomponent filaments, and the multicomponent filaments of the first nonwoven fabric and/or the second nonwoven fabric are crimped by heating so as to increase the volume of the first nonwoven fabric and/or the second nonwoven fabric. This operation typically takes place before the respective nonwoven fabric meets the superabsorbent polymer. Thus, in the case of the first nonwoven fabric, this operation takes place before the superabsorbent polymer is deposited. In the case of the second nonwoven fabric, this operation takes place before laying the second nonwoven fabric on the superabsorbent polymer.

According to a preferred aspect, the filaments are crimped after they have been deposited on the deposit surface on which the nonwoven fabric is formed. Thus, if the source of the first and/or second layer comprises a nonwoven fabric forming apparatus, for example an extrusion apparatus for producing spunbond nonwoven fabric, the filaments are deposited on the deposit surface. Following the deposition, the formed nonwoven fabric is treated, typically heated, so as to crimp the filaments and increase the volume of the nonwoven fabric, and in particular the thickness thereof. The term "source" of the first or second non-woven fabric is intended to define, for example, the means for producing a non-woven fabric in the form of a layer of deposited filaments; a non-woven fabric source may also be a reel of nonwoven fabric.

In case the source of the first and/or second layer comprises an already formed nonwoven fabric, for example a nonwoven fabric, the nonwoven fabric typically has filaments that are not yet crimped, or possibly only partially crimped, so that the nonwoven fabric is treated during the present process, typically heated, so as to crimp the filaments of the respective nonwoven fabric.

Therefore, according to a preferred aspect, during the production of the nonwoven fabric (non-woven fabric) that is subsequently heated, since upon contact with the deposit surface the filaments of this nonwoven fabric are substantially crimp-free, they do not generate a crimp during the steps in which they fall from the spinneret and in which they are heat-treated as they fall.

The difference between non-crimped filaments, i.e., substantially free of crimps, and crimped and non-crimped filaments is known to the person skilled in the art, so that in the non-crimped condition the filaments are substantially free of crimps. Crimped filaments, on the other hand, have a plurality of crimps and a wavy, irregular pattern such that the length of a crimped filament (measured along a straight line and not along the filament path) is significantly less than the length of the same filament in the uncrimped condition. Typically, considering a crimped portion of the filament and considering the distance between the ends (measured along a straight line), said distance is less than 70% of the length of the filament portion measured along the filament, i.e., the distance between the ends measured along a straight line, once this portion has been made substantially straight, without deforming it.

A further definition of crimped filaments is provided, for example, in Reifenhauser Patent Application US20090152757, according to which crimped filaments are considered those having a radius of curvature less than 5 mm in the relaxed state.

Therefore, according to a preferred aspect, the crimping treatment, which is generally a thermal, heating treatment, is carried out after the formation of the nonwoven fabric, i.e., after the deposition of the filaments on a deposit surface.

A significant increase in the thickness of the fabric can be caused by heating the fabric filaments after the deposition of the filaments.

Moreover, it is possible to operate on different parameters of the heat treatment (in particular temperature and duration) in order to obtain the desired result.

According to a preferred aspect, through crimping carried out by heating, the thickness of the nonwoven fabric (non-woven fabric) can be increased between 1.5 and 6 times the initial value (i.e. before heating), while the width and/or length of the nonwoven fabric are reduced by a value between 5% and 40%, preferably between 10% and 30% of the initial value (i.e. the width and/or length of the nonwoven fabric after the heat treatment is equal to or greater than 60% of the respective value before the heat treatment, preferably equal to 70% - 95% of this initial value).

Furthermore, according to a possible aspect, a temperature can be used such that at least one of the materials the filaments are composed of is heated so as to soften at the surface and form mechanical constraints between the filaments.

Crimping the filaments by heating, after the deposition of the filaments themselves and the formation of a layer of nonwoven fabric (even if not subjected to bonding), allows a large particle size of superabsorbent polymer to be retained, and in particular particles with dimensions even smaller than 100 µm, and in particular SAP having particles with dimensions up to 70µm, for example between 70 µm and 100µm (excluded).

The structure thus formed has good mechanical and absorption properties, prevents or reduces the problem of gel blocking of absorbed liquids and is easy to manufacture.

In particular, according to a preferred aspect, at least part of the superabsorbent polymer (SAP) penetrates the first and/or second layers, getting arranged between the multi-component filaments of the nonwoven fabric of the first and/or second layers.

Thus, at least part of the SAP is arranged between the filaments and, according to a possible aspect, substantially all of the SAP is arranged between the filaments of the first and/or second nonwoven fabric.

Typically, due to the heat crimping step, the composite structure is capable of retaining at least 80%, preferably at least 90%, more preferably about 95% of the SAP. In other words, considering a structure portion, in which the layers (excluding SAP) have a total weight X, and considering that this structure portion has been provided with an amount of SAP having weight Y, the final weight of the structure is preferably equal to or greater than X + (0.8 * Y), more preferably equal to or greater than X + (0.9 * Y), even more preferably equal to or greater than X + (0.95 * Y).

The positioning of the superabsorbent polymer between the filaments of the respective nonwoven fabric allows the superabsorbent polymer to be held in place in the end product, thereby preventing the superabsorbent polymer from being dispersed, i.e., escaping from the composite structure. The increased retention in place of the SAP particles allows gel blocking phenomena to be prevented or drastically reduced.

In particular, in nonwoven fabrics in which the filaments have been heat crimped, the superabsorbent polymer particles are more effectively retained by the matrix formed by the gaps between the aforementioned filaments.

It should be noted that the definition of superabsorbent polymer (also known as SAP) is known to the person skilled in the art, since a superabsorbent polymer is typically defined as a polymer capable of absorbing at least 10 times its own weight, preferably at least 15 or 30 times its own weight of a salt solution (0.9% NaCl). Superabsorbent polymers are for example discussed in US2012312491.

According to an aspect, the multicomponent filaments are made of at least two materials that are different from each other at least in terms of physical-mechanical characteristics, the multi-component filaments have at least two sub-filaments bonded with each other and coextruded from said materials, preferably in a side-by-side configuration.

According to a possible aspect, the material of a first sub-filament has a melting temperature different by at least 10°C from the melting temperature of the material of a second sub-filament and, in cross-section, the contact surface between a first sub-filament and a second sub-filament has at least one inflection; the first sub-filament forms at least one protrusion within the second filament, said protrusion having decreasing width.

According to a possible aspect, step d is carried out by a calender having at least one pair of opposed rollers provided with complementary grooves, preferably each roller having two series of helical grooves, which converge towards the center of the roller, each groove series of a roller being complementary to the corresponding series of the other roller.

The superabsorbent polymer is in powder form, and the composite structure is essentially free of cellulose.

According to a possible aspect, the first nonwoven fabric and/or the second nonwoven fabric are/is a nonwoven fabric obtained by spunbond process.

An aspect of the present invention further relates to a composite absorbent structure as obtainable by a process according to one or more of the preceding aspects.

An aspect of the present invention further relates to a composite absorbent structure comprising a first layer comprising a first nonwoven fabric, a second layer comprising a second nonwoven fabric, a superabsorbent polymer arranged at least between the first and second layers, wherein said first nonwoven fabric and/or said second nonwoven fabric is a nonwoven fabric comprising a plurality of crimped multicomponent filaments.

According to a possible aspect, the multicomponent filaments are made of at least two materials different from each other, the multicomponent filaments have at least two sub-filaments coextruded from the materials with a side-by-side arrangement and adhered to one another, and the material of a first sub-filament has a melting temperature different by at least 10°C from the melting temperature of the material of a second sub-filament. In cross-section, the contact surface between a first sub-filament and a second sub-filament has at least one inflection, and the first sub-filament forms at least one protrusion within the second filament, said protrusion having decreasing width.

According to a possible aspect, part of the superabsorbent polymer is arranged between the multicomponent filaments of the first and/or second nonwoven fabric.

According to a possible aspect, the first nonwoven fabric or the second nonwoven fabric, before the heat crimping step, has a first thickness and a first width, and after the heat crimping step and at least before the bonding step has a second thickness and a second width; the second thickness is between 1.5 times and 6 times the first thickness. Preferably, the second width is less than the first width, typically by at least 5% or 10%, and is typically greater than 60% of the first width, most commonly greater than 70%.

An aspect of the present invention further relates to an apparatus for producing a composite absorbent structure according to one or more of the preceding aspects, comprising a deposit surface, a first and a second sources of the first layer and second layer of non-woven fabric, which comprise first and second nonwoven fabrics, respectively, and a third source of the superabsorbent polymer, comprising said superabsorbent polymer that is interposed between first and second sources, so as to deposit the first layer, the superabsorbent polymer and the second layer one upon the other on the support surface, at least one crimping device to crimp by heating the first nonwoven fabric or the second nonwoven fabric, and a calender arranged downstream of the sources.

The term "source" of SAP refers to means adapted to deposit a powdered superabsorbent polymer (SAP) onto the nonwoven fabric.

According to an aspect, the calender has at least one pair of opposed rollers provided with complementary grooves, preferably each roller has two series of helical grooves converging towards the center of the roller, each series of a roller being complementary to the corresponding series of the other roller. Such a shape contributes, among other things, to an optimal distribution of SAP within the composite structure.

According to a possible aspect, the first and/or the second source comprises an extrusion apparatus for producing a spunbond nonwoven fabric (non-woven fabric) or a reel of nonwoven fabric.

According to a possible aspect, the apparatus comprises a device to heat the first nonwoven fabric and/or a device to heat the second nonwoven fabric, before the superabsorbent polymer comes into contact with the first nonwoven fabric and/or the second nonwoven fabric, i.e. before the SAP is fed to the non-woven fabric.

Such a crimping device is typically arranged downstream of the source of the respective nonwoven fabric, so as to heat a nonwoven fabric that has already been at least partially formed by the source itself, i.e. after the filaments have been deposited on a conveyor belt thereof, in a manner known per se in the art.

The absorbent material, preferably powdered SAP, is therefore retained between the two layers comprising the nonwoven fabrics, and preferably also partially between the fibers of the nonwoven fabric of the first and/or second layer, so that the presence of cellulose can be avoided, or at least limited. Typically, the SAP takes an arrangement entirely between the filaments of the first and/or second nonwoven fabric.

In particular, as discussed, it is preferable for the structure to have good mechanical and absorptive properties while allowing a reduction in thickness with equal absorptive capacity.

To this purpose, it is preferred that at least one of the mentioned nonwoven fabrics of the composite structure, or at least one of the mentioned first and second nonwoven fabrics comprises a plurality of filaments made of at least two materials different from each other; the multicomponent filaments have at least two sub-filaments coextruded from the mentioned materials, with a side-by-side arrangement, and are adhered to one another; the material of a first sub-filament has a melting temperature different by at least 10°C from the melting temperature of the material of a second sub-filament. Furthermore, in cross-section, the contact surface between a first sub-filament and a second sub-filament has at least one inflection, and the first sub-filament forms, within the second filament, at least one protrusion having decreasing width. This protrusion gives a wave shape to the contact surface between the two sub-filaments. For the sake of simplicity, therefore, a nonwoven fabric made in this way will be identified hereinafter as a "nonwoven fabric with wave sections". Although such a configuration is the preferred one for the present invention, the latter can also be implemented with different configurations, i.e. the side-by-side ones without wave sections, as well as different configurations such as concentric or eccentric sheath/core.

A nonwoven fabric with wave sections allows the absorbent material, in particular SAP, to be deposited, incorporating the latter between the multicomponent filaments of the former without risk of losses, and forms a composite structure that can be easily processed during the production step, for example by allowing it to be simply wound in reel or folded.

In particular, when a nonwoven fabric with wave sections is heated, its volume (particularly its thickness) increases, due to the different crimping of the sub-filaments comprising the filaments of the nonwoven fabric, as more fully discussed below. According to a possible aspect, such heating may be performed in series with the formation of the nonwoven fabric, or else an unheated nonwoven fabric (or at least heated but not so much as to increase the thickness thereof up to the final value) may be stored, typically wound in reel or folded, so that the thickness increase thereof by heating is carried out (or completed) at a later time, for example in a second plant, possibly arranged in a different location with respect to the production plant of the nonwoven fabric with wave sections.

It should be noted that the first and second layers may consist of the first and second nonwoven fabrics, respectively, or they may comprise additional elements. Typically, the first and second layers comprise only nonwoven fabrics. In other words, the first and second layers preferably consist of a single nonwoven fabric, or are made by means of composite nonwoven fabrics, i.e., the layer comprises sub-layers of nonwoven fabric.

In such a case, at least one of the sub-layers of the first and/or second fabric is typically a nonwoven fabric (non-woven fabric) with crimped filaments, preferably with wave sections.

The composite structure is substantially free of cellulose. Substantially free means that, considering the sum of the weights of superabsorbent polymer and cellulose, the amount of cellulose is less than 15% of this sum, preferably less than 10%. Preferably the amount of cellulose is 0%, or less than 1%.

### Brief description of the figures

Further characteristics and advantages of the present invention will be more evident from the following description made for illustration purposes and without limitation, with reference to the accompanying drawings, wherein:
- figure 1 is a schematic view of the section a composite structure according to an embodiment of the present invention;
- figures 1A and 1B are schematic views of embodiments alternative to those of figure 1;
- figure 2 is a sectional view of possible filaments to form a nonwoven fabric of a layer of a composite structure according to the present invention;
- figure 3 shows sectional views of alternatives of possible filaments to form a nonwoven fabric of a layer of a composite structure according to the present invention;

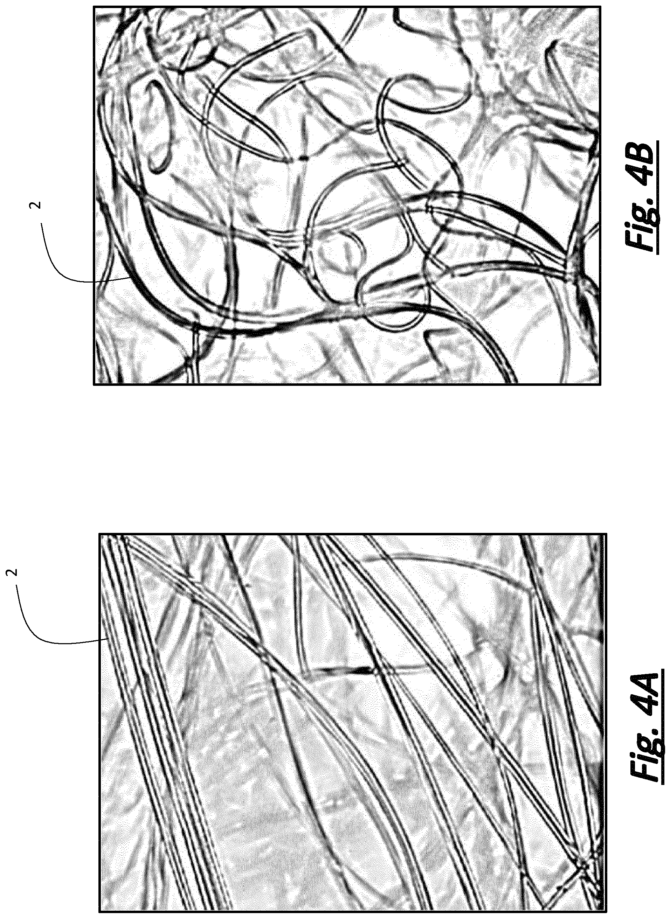

- figures 4A - 4B are enlarged views of a possible nonwoven fabric made with the filaments of figure 2A and *2B*, where the filaments are highlighted before and after the thermal treatment that causes the crimping thereof;

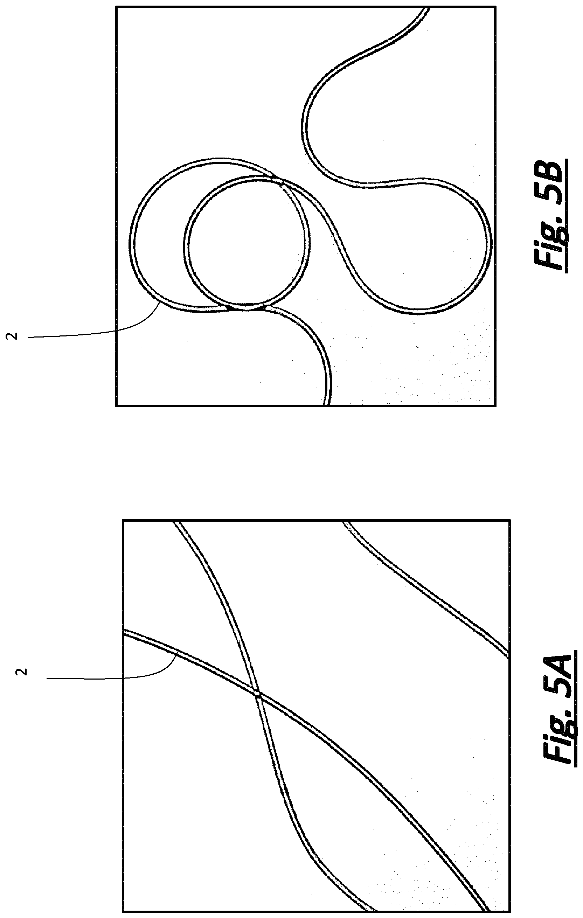

- figures 5A - 5B are enlarged views of individual filaments before and after the thermal treatment causing the crimping thereof;
- figure 6 is a schematic view of an apparatus to make a nonwoven fabric forming part of a layer of a composite structure according to the present invention;
- figures 7 and 8 are schematic views of possible apparatuses for producing a composite structure according to the present invention.

### Detailed description of the figures

Referring to the figures, according to the invention a composite structure 100 comprises a first layer 101 comprising a first nonwoven fabric 101a, a second layer 102 comprising a second nonwoven fabric 102a, and superabsorbent polymer 103 arranged at least between the layers 101, 102. As discussed further below, the superabsorbent polymer 103 may be arranged between the layers 101, 102, but also must at least partially penetrate therein, in particular it has to be arranged between the fibers of the nonwoven fabrics 101a, 102a of the first or second layer 101, 102.

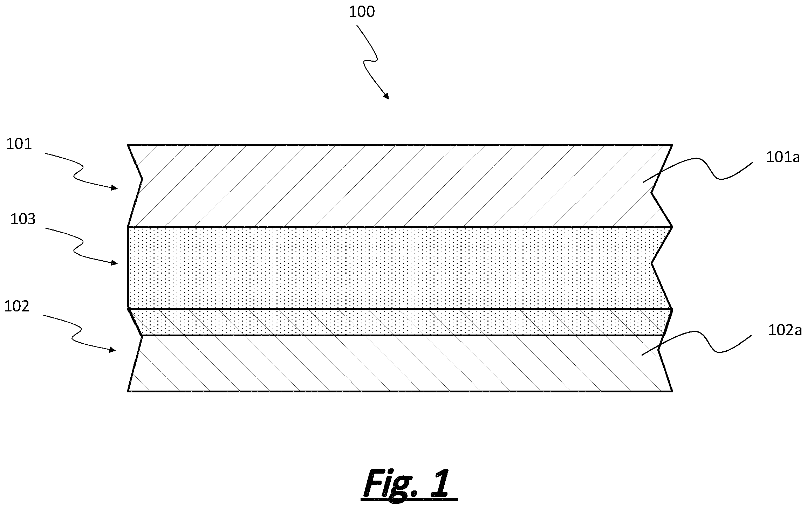

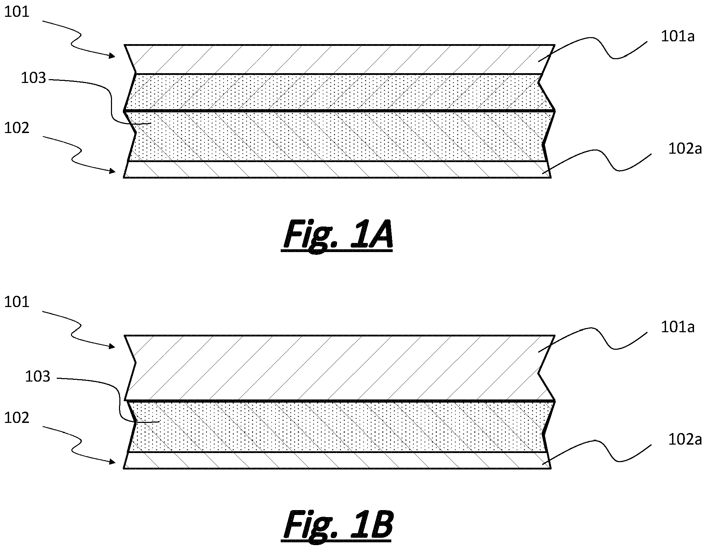

Various solutions are possible. For example, as in the embodiment of figure 1, the superabsorbent polymer 103 may be arranged only partially between the filaments of the nonwoven fabrics of the structure 100, for example only partially between the filaments of the first nonwoven fabric 101a. According to a further preferred embodiment, as in the embodiments of figures 1A and 1B, the superabsorbent polymer may be substantially entirely arranged between the nonwoven fabric filaments of the structure 100. For example, the superabsorbent polymer may get arranged partially between the filaments of the first nonwoven fabric 101a and partially between the filaments of the second nonwoven fabric 102a, as in figure 1A, or it may get arranged between the filaments of a single nonwoven fabric, typically between the filaments of the first nonwoven fabric 101a, as in the exemplified embodiment of figure 1B. Superabsorbent polymers 103 adapted to absorb and retain a liquid are known in the art by the acronym SAP and are not discussed herein in detail. Absorbent materials are typically able to absorb a weight amount of a liquid tens of times their own weight, and typically much larger volumes. For example, superabsorbent polymers able to absorb an amount of (distilled) water that is more than 1000 times their own weight, are known. This property typically decreases depending on any salts or other elements dissolved in the water. It is common in the art to use superabsorbent polymers able to absorb at least 10 times their own weight, preferably at least 30 times their own weight, of a salt solution (0.9% NaCl).

The composite structure 100 is free, or substantially free, of cellulose. In particular, the amount of cellulose coupled to the superabsorbent polymer is preferably less than 15% by weight of the sum of the weights of superabsorbent polymer and cellulose, preferably less than 10%. As discussed above, advantageously the amount of cellulose is 0% by weight of cellulose.

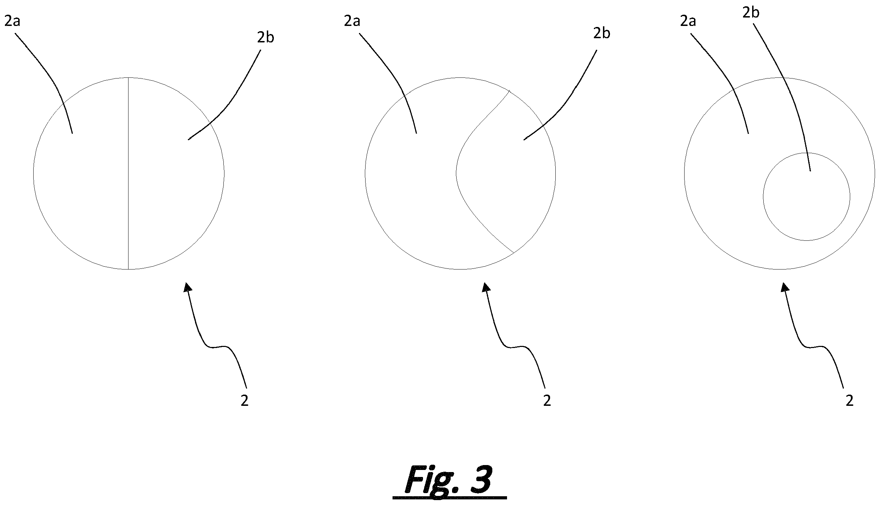

At least one of the first and second layers 101, 102 comprises a nonwoven fabric equipped with multi-component filaments, which are crimped so as to increase the volume of the nonwoven fabric. Preferred shape is the previously mentioned wave shape described herein in detail, although different shapes are possible, some of them schematically shown in figure 3.

Referring to the figures, a nonwoven fabric with crimped filaments T comprises a plurality of multicomponent filaments 2. The multicomponent filaments comprise two sub-filaments 2a, 2b made by co-extruding two typically polymer materials A, B. The sub-filaments 2a, 2b may be arranged according to different configurations, for example side-by-side or core-sheath. Preferred embodiments provide side-by-side configuration.

The materials A, B for the two sub-filaments 2a, 2b are preferably selected among PP, coPP (coPP means PP copolymer), PE, CoPE, PET, CoPET, PA, PLA. Preferred combinations are: PP/PE, PP/PP, PP/coPP, PET/PP, PET/coPET, PLA/PP, PLA/PE, PET/PE. The meaning of these acronyms is known in the art, in particular PP: polypropylene; PE: polyethylene; PET: polyethylene terephthalate; PLA: polylactic acid.

According to a further aspect, the materials are oriented, i.e., non-amorphous, to promote (during a heat treatment) the change in size of the sub-filaments in one direction, so as to allow an increased filament crimp and thus the increase in volume of the nonwoven fabric.

In general, the materials of the sub-filaments 2a, 2b are selected so that the melting temperature T1 of the first sub-filament 2a is different by at least 10°C with respect to the melting temperature T2 of the second filament, more preferably by at least 20°C. In some embodiments, as mentioned above, two different types (i.e., two different "grades") of the same material (for example, two different types of PP) can be used, provided that the condition relating to the difference in melting temperature mentioned above is met.

Preferably, the two materials also have viscosities different from each other, with at least a 20% difference between the two values when measured by the same method. In other words, the difference between the viscosity µ1 of the first sub-filament 2a and the viscosity µ2 of the second filament is more than 20%.

As described above, the two materials (i.e., the materials forming the two sub-filaments 2a, 2b) can be tested with the same viscometer (e.g., rotating or capillary viscometer) or, more generally, the viscosity can be determined by a common method defined in a recognized standard (e.g., ASTM D3835).

Generally, the sub-filament made of material having lower melting temperature also has lower viscosity.

According to a preferred embodiment, the sub-filaments have side-by-side configuration, so that two sub-filaments 2a, 2b result side-by-side to each other. Accordingly, the section of the multicomponent filament 2 has two regions distinct from each other. In other words, the two sub-filaments 2a, 2b, in section, are separated by a line representing the contact surface 3.

The materials A, B of the two sub-filaments 2a, 2b are preferably such that, at the contact surface 3, the sub-filaments 2a, 2b are adhered ("joined") to one another, that is to say the materials are "co-adhesive". Such adherence between the two sub-filaments occurs not only after coextrusion, but also in the final nonwoven fabric. In other words, the treatments carried out on the multicomponent filaments 2 and the end product do not cause the sub-filaments 2a, 2b to separate, i.e. the sub-filaments 2a, 2b are kept adhered to one another.

According to a preferred aspect of the invention, the contact surface 3 has a shape defining at least one protrusion P1 of the first sub-filament 2a within the second sub-filament 2b. As discussed above, the portion of the first sub-filament 2a between the section of the contact surface 3 (i.e., the line defined by the contact surface in cross-section view) and the straight line R passing through the two ends 3a, 3b of the section of the contact surface 3, i.e., the points where the section of the contact surface 3 meets the edge of the filament section 2, will be defined as protrusion P1.

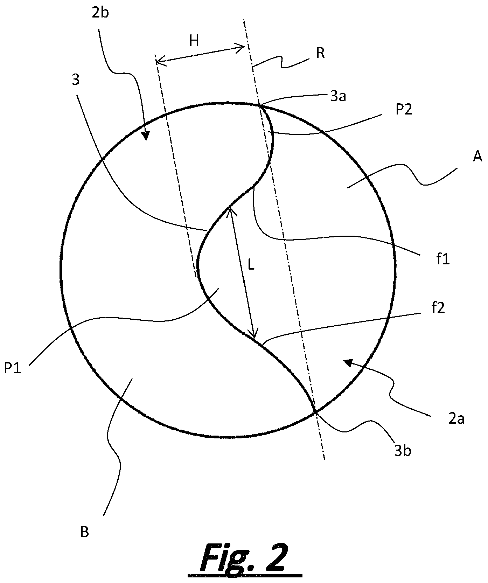

The second sub-filament 2b may also form additional protrusions P2 (a single protrusion P2 is shown in figure 2), i.e., portions of the second filament 2b included between the line R and the section of the contact surface 3. The protrusions P2 are oppositely oriented with respect to the protrusion P1 (i.e., they are arranged on the other side of the straight line R). In a possible embodiment, the protrusions P1, P2 may be specular to each other. In other words, the protrusions P1, P2 may have the same length L and the same height H or similar length and height to each other. In other possible solutions, such as the one shown in the figure, a protrusion P1 may have larger dimensions than the other protrusions P2.

As previously specified, the width L of the protrusions is measured in a direction parallel to the straight line R passing through the two ends 3a, 3b of the section of the contact surface 3, whereas the height H is measured perpendicularly to the width L.

As better discussed below, embodiments in which only a first sub-filament 2a forms a protrusion P1 within the second sub-filament 2b are anyhow possible. Typically, in such embodiments, the straight line R intersects the section of the contact surface 3 only at the ends 3a, 3b thereof.

The contact surface 3 changes at least once its curvature, i.e. has at least one inflection. Preferably, the contact surface 3 (in section) has at least two inflections (or inflection points) f1, f2.

According to an aspect, the inflection points f1, f2 (and more generally the shape of the section of the contact surface 3) are configured to prevent the presence of undercuts.

Actually, a section of the contact surface 3 provided with undercuts is not only complex to implement but also not effective during the crimping step of the filament 2 when, as in the present solution, the two sub-filaments 2a, 2b are adhered to each other.

More details about preferred embodiments having contact surface 3 provided with multiple inflection points f1, f2 can be found in the Application EP3321407 in the name of the Applicant.

Preferably, the multicomponent filament 2 has a yarn count of between 1.5 and 15 den.

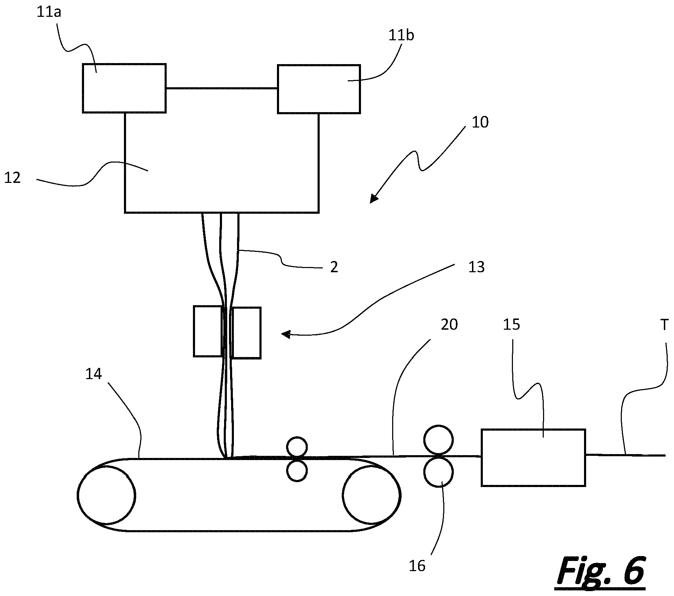

Referring to the illustrative embodiments of figures 6 - 8, an apparatus 10 for producing a nonwoven fabric according to an aspect of the present invention comprises a source 11a of a first material A and a source 11b of a second material B, a spinneret or spinning head 12, drawing means 13, a collecting surface 14 and a heating device 15.

For producing a nonwoven fabric according to the present invention, two polymer materials A, B are provided by the sources 11a, 11b to the spinning head 12. Such polymer materials A, B are the materials the two filaments 2a, 2b are made of.

The two materials A, B are coextruded within the spinneret 12 so as to form a plurality of multicomponent filaments 2. As mentioned, the multicomponent filaments 2 have two sub-filaments 2a, 2b preferably arranged side-by-side and joined by a contact surface 3. According to a preferred embodiment, in section, the contact surface 3 has a shape provided with at least one protrusion P1 as previously described.

Preferably, such a shape is obtained by locally varying the extrusion pressure at different areas of the section of at least one of the sub-filaments 2a, 2b.

Then, the multicomponent filaments 2 are deposited on a collecting surface 14, for example a movable conveyor belt. The filaments get arranged and overlapped substantially randomly on the surface 14 to form a layer 20 of filaments, which are typically overlapped to each other.

In a preferred embodiment, the apparatus 10 is configured such that the filaments are deposited in a substantially non-crimped condition, i.e., they do not develop a crimp as the same filaments fall.

Before deposition, the filaments 2 are drawn by special means 13 known in the art. The apparatus 10 may further comprise means 16, known in the art, for constraining the filaments 2 of the layer 20 to each other at several welding points. For example, in the embodiment shown, there are calenders 16 which can be properly shaped and/or heated in order to facilitate the filaments 2 to be constrained to each other. In any case, alternative means 16 for constraining the filaments 2 are known, for example through needlefelting or by ultrasonic devices. Subsequently, the layer 20 is subjected to a treatment, typically mechanical or thermal, adapted to crimp the filaments 2, by means of a special crimping device 15. Heating is the preferred embodiment, wherein the crimping device 15 is an oven, or otherwise a heating device.

The crimping device 15 is typically arranged downstream of the surface on which the filaments are deposited, and is therefore configured to treat a nonwoven fabric that is already at least partially formed.

Preferably, the layer 20 is heated to a temperature between 100 and 250°C preferably in a period of time between 3 and 50 seconds.

Thanks to the respective treatment, the multicomponent filaments 2 will crimp, so as to increase the thickness of the nonwoven fabric. Alternative embodiments, not shown in detail, may provide, in a known way, mechanical crimping of the multicomponent filaments 2.

In particular, thanks to the treatment of the crimping device 15, particularly in the case of crimping by heating after the filament deposition, an increase of the volume of the nonwoven fabric of between 2 and 5 times with respect to its volume before thermal treatment can be obtained. Typically, crimping occurs by heating, so that during this step a first sub-filament 2a (typically the one having lower melting temperature) shrinks more than the other sub-filament 2b. Since the two sub-filaments are adhered to each other, the first sub-filament 2a bends the second sub-filament 2b, thus causing the second sub-filament 2b to crimp and, as a result, the volume of the nonwoven fabric to increase.

In an alternative embodiment, the filaments of the layer 20 can be simultaneously crimped and constrained to each other by thermal treatment, for example in an oven. In this case, a mechanical constraint caused by crimping and a thermal constraint at contact point caused by high temperature are formed among the filaments.

As above mentioned, during the steps of the above described process, the sub-filaments 2a, 2b remain adhered to one another, so that the filaments 2 are kept intact. In other words, the sub-filaments 2a, 2b of the filaments 2 do not separate (i.e. they do not "split") and are held together in the end product.

Therefore, the obtained nonwoven fabric has low density, and thus is soft and voluminous. Such a nonwoven fabric further has high liquid-absorption capacity.

Preferably, the grammage of the nonwoven fabric with crimped filaments is between 10 and 500 g/m².

As discussed above, the nonwoven fabric with crimped filaments may be used as first nonwoven fabric 101a in the first layer 101, or as second nonwoven fabric 102a in the second layer 102.

In addition, the first and second layers 101, 102 may consist of a single nonwoven fabric, or may be, in turn, composite nonwoven fabrics.

For example, the first layer 101 and/or the second layer 102 may comprise a nonwoven fabric with crimped filaments that is coupled (generally overlapped) to a further nonwoven fabric without crimped filaments.

For the sake of simplicity, an embodiment in which the first and second layers comprise a single nonwoven fabric is described in detail herein, but the present description also applies, mutatis mutandis, to the embodiments in which one or more of the first and second layers are in turn a composite nonwoven fabric.

In general, it is still preferable that first and second layers 101, 102 are only composed of a nonwoven fabric, which may be single, or composite (i.e. provided with two or more sub-layers).

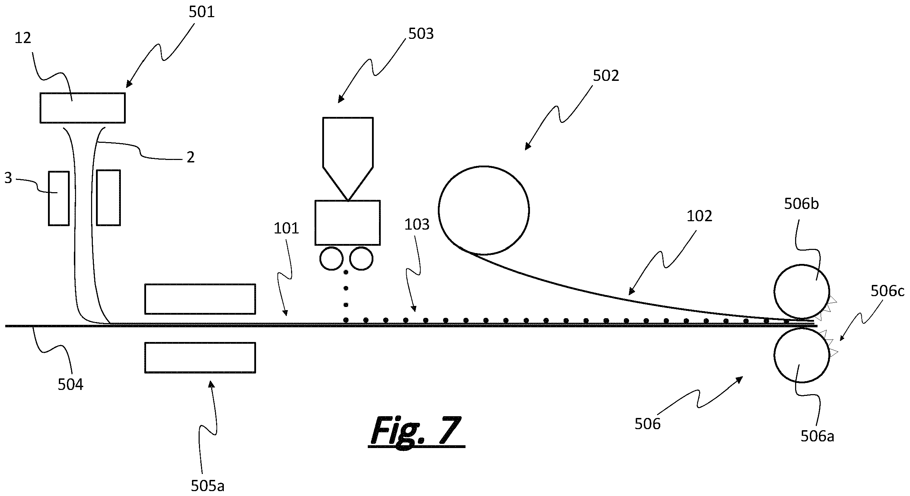

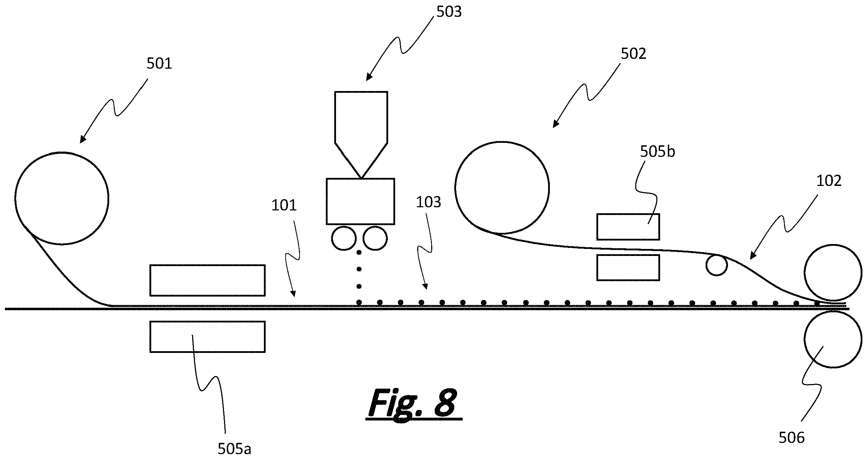

A possible apparatus 10 adapted to produce a nonwoven fabric T with crimped filaments has been shown in figure 6. On the other hand, in figures 7 and 8, possible apparatuses 500 for producing a composite structure 100 are shown.

The apparatus 500 comprises a first source 501 for the first layer 101, a second source for the second layer 502, and a third source 503 for the superabsorbent polymer 103, which is arranged between the first source 501 and the second source 502.

It should be noted that each "source" may comprise multiple elements and it is any element adapted to provide the respective layer. For example, each source may comprise a spinneret, or a reel of nonwoven fabric, for the first and second layers 501, 502 comprising the first nonwoven fabric 101a and the second nonwoven fabric 102a, respectively. As described above, first and second layers may be composite nonwoven fabrics. Therefore, the respective sources may comprise, for example, two different nonwoven fabric reels. In such a case, the respective source is formed by the set of the two nonwoven reels.

In particular, according to a possible aspect, the nonwoven fabric with crimped filaments T may be made directly on this apparatus 500, i.e., the apparatus 500 for producing a composite nonwoven fabric may comprise an apparatus 10 for producing a nonwoven fabric with crimped filaments T.

Such an embodiment is shown in figure 7. According to a possible aspect, therefore, an apparatus 500 for producing a composite nonwoven fabric according to the invention may comprise a deposit surface 504 (typically a movable web), on which the filaments 2 may be deposited for producing a nonwoven fabric with crimped filaments. Similarly to what described above, therefore, the first source 501 of the apparatus 500 has a spinneret or spinning head 12 for extruding the filaments 2, and drawing means 13 to draw them. Typically, there are devices adapted to cool the filaments 2a, 2b.

The deposit surface 504 allows the previously described layer 20 to be formed. Downstream of the first source 501 there is the third source 503 adapted to deposit the superabsorbent polymer 103 on the first layer 101.

Between the first and third sources, there is preferably a crimping device 505a adapted to crimp, by heating, the filaments of the first nonwoven fabric 101a which therefore, in the present case, becomes a nonwoven fabric with crimped filaments T.

The third source 503 is adapted to provide a superabsorbent polymer in powder form, over the previously formed first layer 101. This source, known in the art, is not discussed herein in detail.

Downstream of the third source 503 there is the second source 502 adapted to provide the second layer 102. According to a possible aspect, the second layer 102 comprises a nonwoven fabric 102a that is not a nonwoven fabric with crimped filaments T, and comes from a source 502 in the form of a reel of nonwoven fabric.

It should be noted that, for simplicity, in the case of a source in the form of a reel, a source of nonwoven fabric with crimped filaments T has been discussed. This definition comprises both reels of nonwoven fabric with already crimped filaments and reels of nonwoven fabric with not yet crimped filaments, which may be crimped by a special crimping device arranged downstream of the same source. In particular, the second option is the most common one. It should be noted, however, that even in the case of the first option (i.e. nonwoven fabric with already crimped filaments on reel), said nonwoven fabric is preferably crimped by crimping after the filaments have been deposited in the respective production apparatus of the nonwoven fabric. Such nonwoven fabric was also preferably obtained by deposition of filaments in a substantially uncrimped condition.

The assembly of the layers 101, 102 with the superabsorbent polymer 103 is then typically processed by a calender 506. The calender 506 typically has an operating surface (i.e., the one intended to contact the assembly of layers 101, 102 with the superabsorbent polymer 103) that is not smooth but provided with recesses or protrusions 506c shown in figure 7 only schematically.

According to a preferred aspect, the calender 506 has at least one pair of opposed rollers 506a, 506b provided with complementary grooves 506c. Each roller 506a, 506b preferably has two series of helical grooves converging towards the center of the roller; each series of a roller is complementary to the corresponding series of the other roller. For example, such a calender is described in DE 102018106629.

This calender allows the assembly of the layers 101, 102 with the superabsorbent polymer 103 to be compacted, thus facilitating the composite structure 100 to be formed, and preferably forms holes therein (in particular in the nonwoven fabrics forming this assembly), which increase the absorption properties thereof.

Moreover, the pressure applied by the calender 506 typically forces part of the superabsorbent polymer 103 between the fibers of the nonwoven fabric with crimped filaments T which, as discussed, is present in the first and/or second layers 101, 102. Preferably, a nonwoven fabric with crimped filaments T is arranged at least in the first layer 101 so that not only the action of the calender 506, but also gravity contributes to the distribution of the superabsorbent polymer 103 between the filaments of the nonwoven fabric with crimped filaments T.

As discussed above, the present embodiment may be modified depending on the type of the various layers. A possible variation is shown in figure 8, wherein the nonwoven fabric with crimped filaments T is used in both the first layer 101 and the second layer 102, and wherein both the first source 501 and the second source 502 are in the form of a nonwoven fabric reel. In such a case, the respective nonwoven fabric with crimped filaments was previously formed by means of a designated apparatus (e.g., the apparatus 10 of figure 6), and subsequently wound onto a reel.

Preferably, in such a case, the respective apparatus 10 for producing in advance the nonwoven fabric with crimped filaments is not provided with a crimping device 15 or, alternatively, such a device is configured to perform crimping (e.g., by heating) that does not complete the volume increase of the nonwoven fabric with crimped filaments, for example by a temperature and/or heat exposure time lower than that required to complete the increase in thickness of the nonwoven fabric. As a result, the resulting nonwoven fabric can be wound on reel (or otherwise stored by means of a suitable container or element), while having a reduced thickness compared to the final condition. Such a reel or similar element is subsequently used as a source 501, 502 in the apparatus 500 later used to form the composite structure 100.

Therefore, on this apparatus 500 for forming the composite structure 100, crimping devices 505a, 505b are provided downstream of the first source 501 and the second source 502, so as to perform (or complete) the increase in thickness of the respective nonwoven fabric with crimped filaments, typically by crimping the respective filaments.

Further variations of the present invention, not shown in detail, are possible.

For example, in a variation of the embodiment of figure 7, the first source 501 could comprise a nonwoven fabric reel with crimped filaments T. Furthermore, in a possible variation, the second source 502 could comprise a reel of nonwoven fabric with crimped filaments T and possibly a related crimping device downstream of the reel. In addition, the first source 501 could comprise two different spinnerets, a first spinneret for a nonwoven fabric with crimped filaments, and a second spinneret for a common nonwoven fabric, etc. Similar variations may be provided for the embodiment of figure 8.

In general, the first nonwoven fabric 101a and/or the second nonwoven fabric 102a are preferably produced by a spunbond process, in which the filaments are first deposited and then crimped by heating. Therefore, the filaments are typically deposited in a substantially uncrimped condition, i.e., the filaments are not subjected to treatments that cause them to crimp as they fall from the respective spinneret towards a deposit surface.

In the production step of a composite structure 100 according to the present invention, the first, second and third sources 501, 502, 503 are operated so that the first layer 101 (provided with the first nonwoven fabric 101a) is arranged on a deposit surface 504, the superabsorbent polymer 103 is subsequently arranged on the first layer 101, and the second layer (equipped with the second nonwoven fabric 102a) is subsequently arranged on the assembly of the first layer and superabsorbent polymer 101, 103.

As discussed, these steps may vary depending on the type of layers used.

For example, a step of crimping the filaments of the first and/or second nonwoven fabric 101a, 102a is typically performed before the latter contacts the superabsorbent polymer 103. This step preferably comprises heating to a temperature between 100 and 250°C, preferably in a period of time between 2 and 50 seconds.

Additionally, as discussed, the operation of the sources (particularly the sources 501, 502) of nonwoven fabric may vary in different embodiments. As a matter of fact, such a source may be configured to make the respective nonwoven fabric at that moment (typically by means of a spinneret) or else it may comprise the respective finished or semi-finished nonwoven fabric (e.g. not yet heated). Similarly, the sources 501, 502 may be configured to provide a layer 101, 102 that in turn comprises a composite nonwoven fabric, whereby the sources 501, 502 may be configured so as to produce or provide one or more nonwoven fabrics intended to form the respective layer 101, 102.

## Claims

1. Process for making a composite absorbent structure (100), comprising the steps of:
a. Feeding, from a first source (501) onto a deposit surface (504), a first layer (101) comprising a first nonwoven fabric (101a);
b. Feeding superabsorbent polymer (103) on said first layer (101);
c. Feeding, from a second source onto the assembly formed by said first layer (101) and said superabsorbent polymer (103), a second layer (102) comprising a second nonwoven fabric (102a);
d. Bonding said first nonwoven fabric and said second nonwoven fabric to each other by means of at least one calender (506);
wherein said first nonwoven fabric and/or said second nonwoven fabric comprise/s, preferably is essentially composed of, multicomponent filaments, and wherein said process comprises the step of crimping by heating the multicomponent filaments of said first nonwoven fabric and/or of said second nonwoven fabric so as to increase the volume of said first nonwoven fabric and/or of said second nonwoven fabric, wherein the superabsorbent polymer is in powder form, and the composite structure is essentially free of cellulose.

2. Process according to claim 1, wherein the step of crimping the first and/or second nonwoven fabric comprises the step of crimping the filaments of the first nonwoven fabric (101a) by means of a crimping device (505a) adapted to crimp the filaments of the first nonwoven fabric (101a) by heating so that the first nonwoven fabric (101) becomes a nonwoven fabric with crimped filaments (T), said heating device being arranged between said first source (501) of said first layer and a third source (503) of said superabsorbent polymer (103), wherein said third source (503) is arranged downstream of said first source (501), preferably said heating step comprising heating up to a temperature between 100 and 250°C.

3. Process according to claim 1 or 2, wherein said first and/or said second nonwoven fabric (101a, 102a) is made by a spunbond process, wherein the filaments of said first and/or second nonwoven fabric are crimped by means of a heating device (15, 505a, 505b) after they have been deposited onto a deposit surface.

4. Process according to claim 2 or 3, wherein said first nonwoven fabric or said second nonwoven fabric, before the heat crimping step, has a first thickness and a first width, and after the heat crimping step and before the bonding step, has a second thickness and a second width, said second thickness being between 1.5 times and 6 times the first thickness, preferably said second width is less than the first width, and is greater than 60% of said first width, preferably greater than 70%.

5. Process according to claim 1,2 or 3 wherein in said step b) particles of said superabsorbent polymer have sizes ranging between 70 µm and 100 µm (100 µm being excluded).

6. Process according to claim 1, 2 or 3 wherein said multicomponent filaments are made of at least two materials (A, B) different from each other, said multicomponent filaments (2) comprising at least two sub-filaments (2a, 2b) coextruded from said materials (A, B), preferably with a side-by-side arrangement.

7. Process according to one of the preceding claims, wherein the material of a first sub-filament (2a) has a melting temperature different by at least 10°C from the melting temperature of the material of a second sub-filament (2b) and wherein, in cross-section, the contact surface (3) between a first sub-filament (2a) and a second sub-filament (2b) has at least one inflection point (f1, f2), and wherein the first sub-filament (2a) forms at least one protrusion (P1) within the second filament, said protrusion (P1) having decreasing width.

8. Process according to one of the preceding claims, wherein said step d is carried out by means of a calender (506) having at least one pair of opposed rollers (506a, 506b) provided with complementary grooves (506c), preferably each roller having two series of helical grooves converging towards the center of the roller, each series of a roller being complementary to the corresponding series of the other roller.

9. Composite absorbent structure (100) as achievable by a process according to one or more of the preceding claims.

10. Composite absorbent structure (100) comprising a first layer (101) comprising a first nonwoven fabric (101a), a second layer (102) comprising a second nonwoven fabric (102a), particles of superabsorbent polymer (103) arranged within at least one of said first and second layers (101, 102), wherein said first nonwoven fabric (101a) and/or said second nonwoven fabric (102a) is a nonwoven fabric comprising a plurality of multicomponent filaments (2) crimped by heating themselves, at least part of said particles having sizes ranging between 70 µm and 100µm (100 µm being excluded), wherein the superabsorbent polymer is in powder form, and the composite structure is essentially free of cellulose.

11. Composite absorbent structure (100) according to claim 9 or 10, wherein the multicomponent filaments (2) are made of at least two materials (A, B) different from each other, said multicomponent filaments (2) having at least two sub-filaments (2a, 2b) coextruded from said materials (A, B) with a side-by-side arrangement and adhered to one another, wherein the material of a first sub-filament (2a) has a melting temperature different by at least 10°C from the melting temperature of the material of a second sub-filament (2b) and wherein, in cross-section, the contact surface (3) between a first sub-filament (2a) and a second sub-filament (2b) has at least one inflection (f1, f2), and wherein the first sub-filament (2a) forms at least one protrusion (P1) within the second filament, said protrusion (P1) having decreasing width.

12. Apparatus (500) for producing a composite absorbent structure (100) by means of the process according to one or more of claims 1 to 8, comprising a deposit surface (504), a first and a second source (501, 502) of said first layer (101) and said second layer (102), which comprise said first and second nonwoven fabrics (101a, 102a), respectively, and a third source (503) of superabsorbent polymer (103) interposed between said first and second sources (501, 502), so as to deposit said first layer, said superabsorbent polymer and said second layer (101, 103, 102) one upon the other on said support surface (504), said apparatus (100) comprising at least one crimping device (505a, 505b) to crimp said first nonwoven fabric and/or said second nonwoven fabric by heating, and a calender arranged downstream of said sources (501, 502, 503).

13. Apparatus according to claim 12, wherein said calender has at least one pair of opposed rollers provided with complementary grooves, preferably each roller having two series of helical grooves converging towards the center of the roller, each series of a roller being complementary to the corresponding series of the other roller.

14. Apparatus (500) according to claim 12 or 13, wherein said first and/or said second source (501, 502) comprise/s an extrusion apparatus (10) for the production of a spunbonded nonwoven fabric or a reel of nonwoven fabric.

15. Apparatus according to one of claims 12 to 14, comprising a crimping device (505a) adapted to heat up said first nonwoven fabric (101a) arranged between said first source (501) and said third source (503), said third source (503) being arranged downstream of said first source (501).

## Patentansprüche

1. Verfahren zur Herstellung einer absorbierenden Verbundstruktur (100), umfassend die Schritte:
a. Zuführen einer ersten Schicht (101), die einen ersten Vliesstoff (101a) umfasst, aus einer ersten Quelle (501) auf eine Ablagefläche (504);
b. Zuführen eines superabsorbierenden Polymers (103) auf die erste Schicht (101);
c. Zuführen einer zweiten Schicht (102), die einen zweiten Vliesstoff (102a) umfasst, aus einer zweiten Quelle auf die Anordnung, die aus der ersten Schicht (101) und dem superabsorbierenden Polymer (103) gebildet ist;
d. Verbinden des ersten Vliesstoffs und des zweiten Vliesstoffs miteinander mittels mindestens eines Kalanders (506);
wobei der erste Vliesstoff und/oder der zweite Vliesstoff Mehrkomponentenfilamente umfasst/umfassen, vorzugsweise im Wesentlichen aus diesen besteht/bestehen, und wobei das Verfahren den Schritt des Kräuselns durch Erwärmen der Mehrkomponentenfilamente des ersten Vliesstoffs und/oder des zweiten Vliesstoffs umfasst, so dass das Volumen des ersten Vliesstoffs und/oder des zweiten Vliesstoffs erhöht wird, wobei das superabsorbierende Polymer in einer Pulverform vorliegt und die Verbundstruktur im Wesentlichen frei von Zellulose ist.

2. Verfahren nach Anspruch 1, wobei der Schritt des Kräuselns des ersten und/oder zweiten Vliesstoffes den Schritt des Kräuselns der Filamente des ersten Vliesstoffes (101a) mittels einer Kräuselvorrichtung (505a) umfasst, die dazu ausgebildet ist, die Filamente des ersten Vliesstoffes (101a) durch Erwärmen zu kräuseln, so dass der erste Vliesstoff (101) zu einem Vliesstoff mit gekräuselten Filamenten (T) wird, wobei die Heizvorrichtung zwischen der ersten Quelle (501) der ersten Schicht und einer dritten Quelle (503) des superabsorbierenden Polymers (103) angeordnet ist, wobei die dritte Quelle (503) stromabwärts der ersten Quelle (501) angeordnet ist, wobei der Heizschritt vorzugsweise ein Erwärmen auf eine Temperatur zwischen 100 und 250°C umfasst.

3. Verfahren nach Anspruch 1 oder 2, wobei der erste und/oder der zweite Vliesstoff (101a, 102a) in einem Spinnvliesverfahren hergestellt wird, wobei die Filamente des ersten und/oder zweiten Vliesstoffes nach dem Ablegen auf einer Ablagefläche mittels einer Heizvorrichtung (15, 505a, 505b) gekräuselt werden.

4. Verfahren nach Anspruch 2 oder 3, wobei der erste Vliesstoff oder der zweite Vliesstoff vor dem Wärmekräuselschritt eine erste Dicke und eine erste Breite aufweist und nach dem Wärmekräuselschritt und vor dem Verbindungsschritt eine zweite Dicke und eine zweite Breite aufweist, wobei die zweite Dicke zwischen dem 1,5-fachen und dem 6-fachen der ersten Dicke beträgt, wobei vorzugsweise die zweite Breite kleiner als die erste Breite und größer als 60 % der ersten Breite, vorzugsweise größer als 70 % ist.

5. Verfahren nach Anspruch 1, 2 oder 3, wobei in dem Schritt b) Teilchen des superabsorbierenden Polymers Größen im Bereich von 70 µm bis 100 µm (100 µm ist ausgeschlossen) aufweisen.

6. Verfahren nach Anspruch 1, 2 oder 3, wobei die Mehrkomponentenfilamente aus mindestens zwei voneinander verschiedenen Materialien (A, B) hergestellt sind, wobei die Mehrkomponentenfilamente (2) mindestens zwei aus den Materialien (A, B) koextrudierte Unterfilamente (2a, 2b) umfassen, vorzugsweise in einer nebeneinanderliegenden Anordnung.

7. Verfahren nach einem der vorhergehenden Ansprüche, wobei das Material eines ersten Unterfilaments (2a) eine Schmelztemperatur aufweist, die sich um mindestens 10°C von der Schmelztemperatur des Materials eines zweiten Unterfilaments (2b) unterscheidet, und wobei die Kontaktfläche (3) zwischen einem ersten Unterfilament (2a) und einem zweiten Unterfilament (2b) im Querschnitt mindestens einen Wendepunkt (f1, f2) aufweist, und wobei das erste Unterfilament (2a) innerhalb des zweiten Filaments mindestens eine Ausbuchtung (P1) bildet, wobei die Ausbuchtung (P1) eine abnehmende Breite aufweist.

8. Verfahren nach einem der vorhergehenden Ansprüche, wobei der Schritt d mittels eines Kalanders (506) ausgeführt wird, der mindestens ein Paar gegenüberliegender Walzen (506a, 506b) aufweist, die mit komplementären Rillen (506c) versehen sind, wobei vorzugsweise jede Walze zwei Reihen spiralförmiger Rillen aufweist, die in Richtung der Walzenmitte zusammenlaufen, wobei jede Reihe einer Walze die entsprechende Reihe der anderen Walze komplimentiert.

9. Absorbierende Verbundstruktur (100), erhältlich durch ein Verfahren gemäß einem oder mehreren der vorhergehenden Ansprüche.

10. Absorbierende Verbundstruktur (100), die eine erste Schicht (101) mit einem ersten Vliesstoff (101a), eine zweite Schicht (102) mit einem zweiten Vliesstoff (102a) und Teilchen aus superabsorbierendem Polymer (103) umfasst, die in mindestens einer der ersten und zweiten Schichten (101, 102) angeordnet sind, wobei der erste Vliesstoff (101a) und/oder der zweite Vliesstoff (102a) ein Vliesstoff ist, der eine Vielzahl von durch Erwärmen gekräuselten Mehrkomponentenfilamenten (2) umfasst, wobei mindestens ein Teil der Teilchen Größen im Bereich zwischen 70 µm und 100 µm aufweist (100 µm ist ausgeschlossen), wobei das superabsorbierende Polymer in einer Pulverform vorliegt und die Verbundstruktur im Wesentlichen frei von Zellulose ist.

11. Absorbierende Verbundstruktur (100) nach Anspruch 9 oder 10, wobei die Mehrkomponentenfilamente (2) aus mindestens zwei voneinander verschiedenen Materialien (A, B) hergestellt sind, wobei die Mehrkomponentenfilamente (2) mindestens zwei Subfilamente (2a, 2b) aufweisen, die aus den Materialien (A, B) in einer Seite-an-Seite-Anordnung koextrudiert worden und aneinander haftend sind, wobei das Material eines ersten Unterfilaments (2a) eine Schmelztemperatur aufweist, die sich um mindestens 10°C von der Schmelztemperatur des Materials eines zweiten Unterfilaments (2b) unterscheidet, und wobei die Kontaktoberfläche (3) zwischen einem ersten Unterfilament (2a) und einem zweiten Unterfilament (2b) im Querschnitt mindestens eine Biegung (f1, f2) aufweist, und wobei das erste Unterfilament (2a) innerhalb des zweiten Filaments mindestens einen Vorsprung (P1) bildet, wobei dieser Vorsprung (P1) eine abnehmende Breite aufweist.

12. Vorrichtung (500) zur Herstellung einer absorbierenden Verbundstruktur (100) mittels des Verfahrens gemäß einem oder mehreren der Ansprüche 1 bis 8, umfassend eine Ablagefläche (504), eine erste und eine zweite Quelle (501, 502) der ersten Schicht (101) und der zweiten Schicht (102), die jeweils den ersten und den zweiten Vliesstoff (101a, 102a) umfassen, und eine dritte Quelle (503) eines superabsorbierenden Polymers (103), die zwischen der ersten und der zweiten Quelle (501, 502) angeordnet ist, um die erste Schicht, das superabsorbierende Polymer und die zweite Schicht (101, 103, 102) übereinander auf der Trägerfläche (504) abzulegen, wobei die Vorrichtung (100) mindestens eine Kräuselvorrichtung (505a, 505b) zum Kräuseln des ersten Vliesstoffs und/oder des zweiten Vliesstoffs, vorzugsweise durch Erwärmen, und einen Kalander umfasst, der stromabwärts der Quellen (501, 502, 503) angeordnet ist.

13. Vorrichtung nach Anspruch 12, wobei der Kalander mindestens ein Paar gegenüberliegender Walzen mit komplementären Rillen aufweist, wobei vorzugsweise jede Walze zwei Reihen spiralförmiger Rillen aufweist, die zur Walzenmitte hin zusammenlaufen, wobei jede Reihe einer Walze die entsprechende Reihe der anderen Walze komplimentiert.

14. Vorrichtung (500) nach Anspruch 12 oder 13, wobei die erste und/oder die zweite Quelle (501, 502) eine Extrusionsvorrichtung (10) zur Herstellung eines Spinnvlieses oder einer Vliesrolle umfasst.

15. Vorrichtung nach einem der Ansprüche 12 bis 14, die eine zum Erwärmen des ersten Vliesstoffs (101a) ausgebildete Kräuselvorrichtung (505a) aufweist, die zwischen der ersten Quelle (501) und der dritten Quelle (503) angeordnet ist, wobei die dritte Quelle (503) stromabwärts der ersten Quelle (501) angeordnet ist.

## Revendications

1. Procédé de fabrication d'une structure absorbante composite (100), comprenant les étapes consistant à :
a. alimenter, à partir d'une première source (501) sur une surface de dépôt (504), une première couche (101) comprenant un premier tissu non tissé (101a) ;
b. alimenter en polymère superabsorbant (103) ladite première couche (101) ;
c. alimenter, à partir d'une deuxième source, sur l'ensemble formé par ladite première couche (101) et ledit polymère superabsorbant (103), une deuxième couche (102) comprenant un deuxième tissu non tissé (102a) ;
d. coller ledit premier tissu non tissé et ledit deuxième tissu non tissé l'un à l'autre au moyen d'au moins une calandre (506) ;
dans lequel ledit premier tissu non tissé et/ou ledit deuxième tissu non tissé comprend/comprennent, de préférence est essentiellement composé de filaments multicomposants, et dans lequel ledit procédé comprend l'étape de sertissage par chauffage des filaments multicomposants dudit premier tissu non tissé et/ou dudit deuxième tissu non tissé de manière à augmenter le volume dudit premier tissu non tissé et/ou dudit deuxième tissu non tissé, dans lequel le polymère superabsorbant est sous forme de poudre, et la structure composite est essentiellement dépourvue de cellulose.

2. Procédé selon la revendication 1, dans lequel l'étape de sertissage du premier et/ou du deuxième tissu non tissé comprend l'étape de sertissage des filaments du premier tissu non tissé (101a) au moyen d'un dispositif de sertissage (505a) adapté pour sertir les filaments du premier tissu non tissé (101a) par chauffage de sorte que le premier tissu non tissé (101) devienne un tissu non tissé à filaments sertis (T), ledit dispositif de chauffage étant disposé entre ladite première source (501) de ladite première couche et une troisième source (503) dudit polymère superabsorbant (103), dans lequel ladite troisième source (503) est disposée en aval de ladite première source (501), de préférence ladite étape de chauffage comprenant le chauffage jusqu'à une température comprise entre 100 et 250°C.

3. Procédé selon la revendication 1 ou 2, dans lequel ledit premier et/ou ledit deuxième tissu non tissé (101a, 102a) est fabriqué par un procédé filé-lié, dans lequel les filaments dudit premier et/ou deuxième tissu non tissé sont sertis au moyen d'un dispositif de chauffage (15, 505a, 505b) après qu'ils ont été déposés sur une surface de dépôt.

4. Procédé selon la revendication 2 ou 3, dans lequel ledit premier tissu non tissé ou ledit deuxième tissu non tissé, avant l'étape de sertissage à chaud, a une première épaisseur et une première largeur, et après l'étape de sertissage à chaud et avant l'étape de collage, a une deuxième épaisseur et une deuxième largeur, ladite deuxième épaisseur étant comprise entre 1,5 fois et 6 fois la première épaisseur, de préférence ladite deuxième largeur est inférieure à la première largeur, et est supérieure à 60 % de ladite première largeur, de préférence supérieure à 70 %.

5. Procédé selon la revendication 1, 2 ou 3 dans lequel, dans ladite étape b), les particules dudit polymère superabsorbant ont des tailles comprises entre 70 µm et 100 µm (100 µm étant exclu).

6. Procédé selon la revendication 1, 2 ou 3 dans lequel lesdits filaments multicomposants sont constitués d'au moins deux matériaux (A, B) différents l'un de l'autre, lesdits filaments multicomposants (2) comprenant au moins deux sous-filaments (2a, 2b) coextrudés à partir desdits matériaux (A, B), de préférence avec une disposition côte à côte.

7. Procédé selon l'une quelconque des revendications précédentes, dans lequel le matériau d'un premier sous-filament (2a) a une température de fusion différente d'au moins 10°C de la température de fusion du matériau d'un deuxième sous-filament (2b) et dans lequel, en coupe transversale, la surface de contact (3) entre un premier sous-filament (2a) et un deuxième sous-filament (2b) présente au moins un point d'inflexion (f1, f2), et dans lequel le premier sous-filament (2a) forme au moins une protubérance (P1) dans le deuxième filament, ladite protubérance (P1) ayant une largeur décroissante.

8. Procédé selon l'une des revendications précédentes, dans lequel ladite étape d est réalisée au moyen d'une calandre (506) ayant au moins une paire de rouleaux opposés (506a, 506b) pourvus de rainures complémentaires (506c), de préférence chaque rouleau ayant deux séries de rainures hélicoïdales convergeant vers le centre du rouleau, chaque série d'un rouleau étant complémentaire de la série correspondante de l'autre rouleau.

9. Structure absorbante composite (100) réalisable par un procédé selon une ou plusieurs des revendications précédentes.

10. Structure absorbante composite (100) comprenant une première couche (101) comprenant un premier tissu non tissé (101a), une deuxième couche (102) comprenant un deuxième tissu non tissé (102a), des particules de polymère superabsorbant (103) disposées dans au moins une desdites première et deuxième couches (101, 102), dans lequel ledit premier tissu non tissé (101a) et/ou ledit deuxième tissu non tissé (102a) est un tissu non tissé comprenant une pluralité de filaments multicomposants (2) sertis en se chauffant, au moins une partie desdites particules ayant des tailles comprises entre 70 µm et 100µm (100 µm étant exclu), dans lequel le polymère superabsorbant est sous forme de poudre, et la structure composite est essentiellement dépourvue de cellulose.

11. Structure absorbante composite (100) selon la revendication 9 ou 10, dans laquelle les filaments multicomposants (2) sont constitués d'au moins deux matériaux (A, B) différents l'un de l'autre, lesdits filaments multicomposants (2) ayant au moins deux sous-filaments (2a, 2b) coextrudés à partir desdits matériaux (A, B) avec une disposition côte à côte et collés l'un à l'autre, dans lequel le matériau d'un premier sous-filament (2a) a une température de fusion différente d'au moins 10°C de la température de fusion du matériau d'un deuxième sous-filament (2b) et dans lequel, en coupe transversale, la surface de contact (3) entre un premier sous-filament (2a) et un deuxième sous-filament (2b) présente au moins une inflexion (f1, f2), et dans lequel le premier sous-filament (2a) forme au moins une protubérance (P1) dans le deuxième filament, ladite protubérance (P1) ayant une largeur décroissante.

12. Appareil (500) pour la fabrication d'une structure absorbante composite (100) par le procédé selon une ou plusieurs des revendications 1 à 8, comprenant une surface de dépôt (504), une première et une deuxième source (501, 502) de ladite première couche (101) et de ladite deuxième couche (102), qui comprennent respectivement lesdits premier et deuxième tissus non tissés (101a, 102a), et une troisième source (503) de polymère superabsorbant (103) interposée entre lesdites première et deuxième sources (501, 502), de manière à déposer ladite première couche, ledit polymère superabsorbant et ladite deuxième couche (101, 103, 102) l'un sur l'autre sur ladite surface de support (504), ledit appareil (100) comprenant au moins un dispositif de sertissage (505a, 505b) pour sertir ledit premier tissu non tissé et/ou ledit deuxième tissu non tissé par chauffage, et une calandre disposée en aval desdites sources (501, 502, 503).

13. Appareil selon la revendication 12, dans lequel ladite calandre a au moins une paire de rouleaux opposés pourvus de rainures complémentaires, de préférence chaque rouleau ayant deux séries de rainures hélicoïdales convergeant vers le centre du rouleau, chaque série d'un rouleau étant complémentaire de la série correspondante de l'autre rouleau.

14. Appareil (500) selon la revendication 12 ou 13, dans lequel ladite première et/ou ladite deuxième source (501, 502) comprend un appareil d'extrusion (10) pour la production d'un tissu non tissé filé-lié ou d'une bobine de tissu non tissé.

15. Appareil selon l'une des revendications 12 à 14, comprenant un dispositif de sertissage (505a) adapté pour chauffer ledit premier tissu non tissé (101a) disposé entre ladite première source (501) et ladite troisième source (503), ladite troisième source (503) étant disposée en aval de ladite première source (501).
